# EUROPÄISCHE PATENTSCHRIFT

(11) **EP 1 910 319 B1**
(45) Veröffentlichungstag und Bekanntmachung des Hinweises auf die Patenterteilung: **08.06.2011**
(21) Anmeldenummer: 06777819.1
(22) Anmeldetag: 18.07.2006
(51) Int. Cl.: C07D 265/16, C07D 498/04, C10L 1/22

(54) **VERWENDUNG VON TETRAHYDROBENZOXAZINEN ALS ANTIOXIDANTIEN**
USE OF TETRAHYDROBENZOXAZINES AS ANTIOXIDANTS
UTILISATION DE TETRAHYDROBENZOXAZINES COMME ANTIOXYDANTS

(30) Priorität: 26.07.2005 DE 102005035527
(43) Veröffentlichungstag der Anmeldung: 16.04.2008
(60) Teilanmeldung: 10167054.5
(73) Patentinhaber: BASF SE, 67056 Ludwigshafen (DE)
(72) Erfinder: LANGE, Arno, 67098 Bad Dürkheim (DE); MACH, Helmut, 69115 Heidelberg (DE); RATH, Hans, Peter, 67269 Grünstadt (DE); POSSELT, Dietmar, 69120 Heidelberg (DE)
(86) Internationale Anmeldenummer: PCT/EP2006/064352
(87) Internationale Veröffentlichungsnummer: WO 2007/012580

(56) Entgegenhaltungen:
- EP-A- 1 621 566
- EP-A1- 0 647 700
- EP-A2- 0 811 672
- WO-A-01/25293
- WO-A-01/34581
- WO-A-89/05803
- WO-A2-03/106595
- FR-A- 2 091 605
- GB-A- 2 308 849
- JP-A- 63 149 646
- JP-A- 2003 255 487
- US-A- 3 825 538
- US-A- 4 207 104
- US-A- 4 585 728
- US-A1- 2006 196 107
- FIELDS D L ET AL: "Mannich-type Condensation of Hydroquinone, Formaldehyde and Primary amines" JOURNAL OF ORGANIC CHEMISTRY, AMERICAN CHEMICAL SOCIETY. EASTON, US, Bd. 27, 1962, Seiten 2749-2753, XP002228613 ISSN: 0022-3263
- HO-DONG KIM ET AL.: "Model compounds study on the network structure of polybenzoxazines" MACROMOLECULES., Bd. 36, 2003, Seiten 8320-8329, XP002404381 USAMERICAN CHEMICAL SOCIETY, EASTON, PA.
- W. J. BURKE ET AL.: "Mon-1,3-benzoxazines from hydroquinone" JOURNAL OF ORGANIC CHEMISTRY., Bd. 28, 1963, Seiten 1098-1100, XP002404382 USAMERICAN CHEMICAL SOCIETY, WASHINGTON, DC.
- DELBERT D. REYNOLDS ET AL.: "1,3,5-Trisubstituted hexahydrotriazines as Mannich reagents." JOURNAL OF HETEROCYCLIC CHEMISTRY, Bd. 8, 1971, Seiten 611-615, XP002404383 USPROVO, UT
- PAUL D. WOODGATE ET AL.: "Synthesis of dioxazaborocines...." JOURNAL OF ORGANOMETALLIC CHEMISTRY., Bd. 592, 1999, Seiten 180-193, XP002404384 CHELSEVIER SEQUOIA S.A., LAUSANNE.

## Beschreibung

Die vorliegende Erfindung betrifft die Verwendung von speziellen Tetrahydrobenzoxazinen als Antioxidantien zur Stabilisierung von Mineralölprodukten und Kraftstoffen gegen die Einwirkung von Licht, Sauerstoff und Wärme, insbesondere in Turbinenkraftstoffen (jet fuels). Weiterhin betrifft die vorliegende Erfindung eine Turbinenkraftstoffzusammensetzung und ein Additivkonzentrat für Turbinenkraftstoffe, welche diese Tetrahydrobenzoxazine enthalten.

Die mechanischen, chemischen und/oder ästhetischen Eigenschaften von Mineralölprodukten und Kraftstoffen werden bekanntermaßen durch die Einwirkung von Licht, Sauerstoff und Wärme verschlechtert. Diese Verschlechterung zeigt sich üblicherweise als Vergilbung oder Verfärbung des Materials. Es sind schon Stabilisatoren oder Stabilisatorzusammensetzungen bekannt, mit denen ein verbesserter Schutz gegen eine solche Beeinträchtigung von organischem Material durch Licht, Sauerstoff und Wärme erzielt werden kann.

So werden in der WO 05/073152 (1) 2-Alkyl-polyisobutenylphenole und deren Mannich-Addukte als Antioxidantien zur Stabilisierung von unbelebtem organischen Material gegen die Einwirkung von Licht, Sauerstoff und Wärme beschrieben. Als zu stabilisierende Materialien werden auch Kraftstoffe wie Ottokraftstoffe, Dieselkraftstoffe und Turbinenkraftstoffe sowie Schmierstoffzusammensetzungen genannt. In Turbinenkraftstoffen bewirken diese 2-Alkyl-polyisobutenyl-phenole und deren Mannich-Addukte eine Verbesserung der Thermostabilität sowie eine Verringerung der Ablagerungen im Kraftstoffkreislauf und Verbrennungssystem der Turbinen.

Auch die WO 03/106595 (2) offenbart neben hydrocarbylsubstituierten Bernsteinsäurederivaten und Polyalkenylthiophosphonatestern Mannich-Addukte aus hydrocarbylsubstituierten Phenolen, einem Aldehyd und einem Amin als Additive für Turbinenkraftstoffe (jet fuels) zur Verbesserung der Thermostabilität sowie zur Verringerung von Ablagerungen.

In der US 2 458 526 werden Kondensationsprodukte von 8-Hydroxychinolin mit einem Aldehyd und Ammoniak oder einem Amin als multifunktionelle Additive für Mineralöle beschrieben. Diese Kondensationsprodukte enthalten einen an einem Tetrahydrooxazin-Ring ankondensierten Chinolin-Ring.

Es besteht jedoch für den Mineralölprodukte- und Kraftstoff-Bereich ein Bedarf an Antioxidantien mit verbesserter Schutzwirkung gegen die Beeinträchtigung der Materialeigenschaften durch Licht, Sauerstoff und Wärme. Vor allem für Turbinenkraftstoffe (jet fuels), die einer extremen thermischen Belastung beim und vor dem Verbrennungsvorgang in Turbinen, beispielsweise in Flugzeugturbinen, ausgesetzt sind, werden neue verbesserte Antioxidantien gesucht. Diese sollen in den Turbinen gleichzeitig über ihre Wirkungsweise als Antioxidantien und/oder Dispergatoren auch Ablagerungen im Kraftstoffkreislauf und im Verbrennungssystem verringern.

Es bestand daher die Aufgabe, Antioxidantien mit einer verbesserten Stabilisierung von Mineralölprodukten und Kraftstoffen, vor allem von Turbinenkraftstoff, gegen die Einwirkung von Licht, Sauerstoff und Wärme bereitzustellen.

Demgemäß wurde die Verwendung von Tetrahydrobenzoxazinen der allgemeinen Formel I 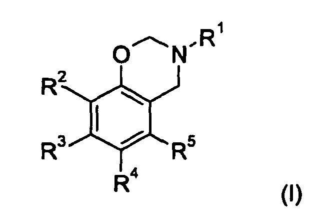 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und
die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen stehen, welche durch ein oder mehrere Heteroatome aus der Gruppe O und S und/oder durch eine oder mehrere Gruppierungen NR⁶ unterbrochen sein können,
wobei der Substituent R⁴ auch für einen Rest der Formel Y stehen kann 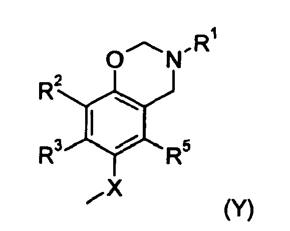 in dem die Substituenten R¹, R², R³ und R⁵ die vorgenannten Bedeutungen haben und der Substituent X ein Kohlenwasserstoff-Brückenglied bezeichnet, welches aus einem oder mehreren Isobuten-Einheiten besteht oder ein oder mehrere Isobuten-Einheiten enthält, oder
wobei der Substituent R⁴ auch für einen Rest der Formel Z oder Z' stehen kann 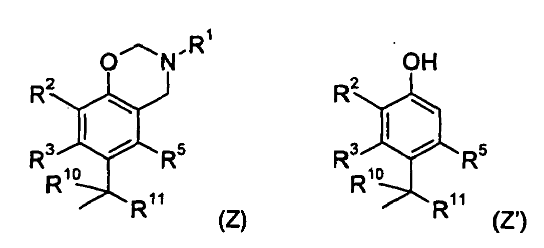 in denen die Substituenten R¹, R², R³ und R⁵ die vorgenannten Bedeutungen haben und die Substituenten R¹⁰ und R¹¹ gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR⁷-CH₂- auch einen zweiten Tetrahydrobenzoxazin-Ring oder die Substituenten R² und R³ und R⁴ und R⁵ mit den an den Benzolkern angebundenen Teilstrukturen -O-CH₂-NR⁷-CH₂- und -O-CH₂-NR⁸-CH₂-auch einen zweiten und einen dritten Tetrahydrobenzoxazin-Ring ausbilden können,
wobei R⁷ und R⁸ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können,
mit der Maßgabe, dass mindestens einer der Substituenten R¹, R², R³, R⁴, R⁵, R⁷ oder R⁸ 4 bis 3000 Kohlenstoffatome aufweist und die übrigen Substituenten aus der Gruppe R¹, R², R³, R⁴, R⁵, R⁷ und R⁸, wenn sie für Hydrocarbylreste stehen, jeweils 1 bis 20 Kohlenstoffatome aufweisen,
als Antioxidantien zur Stabilisierung von Mineralölprodukten und Kraftstoffen gegen die Einwirkung von Licht, Sauerstoff und Wärme gefunden.

Tetrahydrobenzoxazine sind im Prinzip als Zusatzstoffe für Kraftstoff und Schmierstoffzusammensetzungen bekannt. So offenbaren die WO 01/25293 (3) und die WO 01/25294 (4) Tetrahydrobenzoxazine mit längerkettigen Resten wie Polyisobutenylresten, welche als Substituenten am Benzolkern sitzen, als ventilreinigende und ventilreinhaltende Ottokraftstoffdetergentien. Diese Tetrahydrobenzoxazine werden gemäß den in (3) und (4) genannten Herstellverfahren als Gemische mit den entsprechenden offenkettigen Mannich-Addukten des zugrundeliegenden Phenols erhalten und auch so in den Ottokraftstoffen eingesetzt.

Die Herstellung von Tetrahydrobenzoxazinen mit kurzkettigen Substituenten, welche sich als Pflanzenschutzmittel eignen, aus 4-Alkylphenolen und einem Addukt aus beispielsweise 1 Mol Cyclohexylamin und 2 Mol Formaldehyd oder Paraformaldehyd in Methanol oder Ethanol als Lösungsmittel ist in der US-A 2 806 031 (5) und der US-A 3 132 960 (6) beschrieben.

Die strukturelle Besonderheit der erfindungsgemäß zu verwendenden Tetrahydrobenzoxazine I ist, dass sie mindestens einen längerkettigen Hydrocarbylrest mit 4 bis 3000 Kohlenstoffatomen als einen der Substituenten R¹, R², R³, R⁴, R⁵, R⁷ oder R⁸ entweder am Benzolkern oder an einem Oxazinring enthalten. In einer bevorzugten Ausführungsform ist dieser längerkettige Hydrocarbylrest mit 4 bis 3000 Kohlenstoffatomen ein Polyisobutenylrest. Der genannte längerkettige Hydrocarbylrest kann in einer weiteren bevorzugten Ausführungsform auch einen C₁₆- bis C₂₀-Alkyl- oder Alkenylrest bedeuten. Insbesondere sitzt dieser längerkettige Hydrocarbylrest, der vorzugsweise ein Polyisobutenylrest oder ein C₁₆- bis C₂₀-Alkyl- oder Alkenylrest ist, an einem Oxazinring, d.h. er tritt als Substituent R¹ oder R⁷ oder R⁸ auf. Vorzugsweise sitzt dieser längerkettige Hydrocarbylrest, der vorzugsweise ein Polyisobutenylrest oder ein C₁₆- bis C₂₀-Alkyl- oder Alkenylrest ist, auch am Benzolkern als Substituent R² oder R⁴. Dieser längerkettige Hydrocarbylrest, der vorzugsweise ein Polyisobutenylrest oder ein C₁₆- bis C₂₀-Alkyl- oder Alkenylrest ist, umfasst vorzugsweise 16 bis 3000, insbesondere 20 bis 1000, vor allem 25 bis 500, ganz besonders bevorzugt 30 bis 250 Kohlenstoffatome. Im Falle von Polyisobutenylresten weisen diese zahlenmittlere Molekulargewichte Mₙ von 200 bis 40.000, vorzugsweise 500 bis 15.000, insbesondere 700 bis 7000, vor allem 900 bis 3000, ganz besonders bevorzugt 900 bis 1100 auf.

Als C₁₆- bis C₂₀-Alkyl- oder Alkenylreste eignen sich zweckmäßigerweise die Reste von entsprechenden gestättigten oder ungesättigten Fettalkoholen mit 16 bis 20 Kohlenstoffatomen. Insbesondere sind hier n-Hexadecyl (Palmityl), n-Octadecyl (Stearyl), n-Eicosyl, Oleyl, Linolyl und Linolenyl zu nennen, welche gemäß ihrem natürlichen Vorkommen meist als technische Gemische untereinander auftreten.

Der genannte längerkettige Hydrocarbylrest mit 4 bis 3000 Kohlenstoffatomen kann in den Tetrahydrobenzoxazinen I auch mehrfach, beispielsweise zweifach oder dreifach, vertreten sein. Dieser längerkettige Hydrocarbylrest, der vorzugsweise ein Polyisobutenylrest und/oder ein C₁₆- bis C₂₀-Alkyl- oder Alkenylrest ist, tritt bei zweifachem Auftreten beispielsweise als Substituent R¹ und R⁴ oder R¹ und R⁷ auf.

In einer bevorzugten Ausführungsform treten ein oder zwei Polyisobutenyl-Reste mit einem zahlenmittleren Molekulargewicht Mₙ von 200 bis 40.000 im Molekül als Substituent R¹ und/oder R² und/oder R⁴ und/oder R⁷ und/oder R⁸ auf.

Die übrigen Substituenten aus der Gruppe R¹, R², R³, R⁴, R⁵, R⁷ und R⁸, die nicht für Substituenten mit 4 bis 3000 Kohlenstoffatomen oder für Polyisobutenylreste mit einem zahlenmittleren Molekulargewichte Mₙ von 200 bis 40.000 stehen, bezeichnen unabhängig voneinander Wasserstoffatome, Hydroxylgruppen oder, wenn sie für Hydrocarbylreste stehen, meist kürzerkettige Hydrocarbylreste mit 1 bis 20, vorzugsweise 1 bis 12, vor allem 1 bis 8, ganz besonders bevorzugt lineare oder verzweigte C₁- bis C₄-Alkylreste. Typische Beispiele für die letzteren sind Methyl, Ethyl, n-Propyl, iso-Propyl, n-Butyl, 2-Butyl, sec.-Butyl und tert.-Butyl. Methylreste und tert.-Butylreste werden hierbei ganz besonders bevorzugt.

Bevorzugt erfindungsgemäß einzusetzende Tetrahydrobenzoxazine I sind auch solche, bei denen die Substituenten R² und/oder R⁴, wenn sie für kürzerkettige Hydrocarbylreste stehen, lineare oder verzweigte C₁- bis C₄-Alkylreste, insbesondere Methylreste und/oder tert.-Butylreste, bezeichnen. Derartige Substitutionsmuster kommen natürlich nur für Tetrahydrobenzoxazine I mit insgesamt einem oder zwei Tetrahydrooxazin-Ringsystemen in Betracht.

Im Rest der Formel Y bezeichnet der Substituent X ein Kohlenwasserstoff-Brückenglied, welches aus einem oder mehreren, vorzugsweise 4 bis 800, insbesondere 10 bis 300, vor allem 12 bis 100 Isobuten-Einheiten besteht oder ein oder mehrere, vorzugsweise 4 bis 800, insbesondere 10 bis 300, vor allem 12 bis 100 Isobuten-Einheiten enthält. Besteht X aus Isobuten-Einheiten, erfolgt die Verknüpfung in der Relgel über die das α- und das ω-Kohlenstoffatom. Enthält X weitere Kohlenwasserstoff-Baueinheiten, sind dies vorzugsweise mittenständig angeordnete Initiatormolekül-Baueinheiten wie aromatische Ringsysteme, beispielsweise o-, m- oder p-Phenylen-Einheiten, und/oder Kohlenwasserstoff-Baueinheiten mit funktionellen Gruppen zur Verknüpfung, beispielsweise o-, m- oder p-Hydroxyphenyl-Gruppen, als beidseitiger Kettenabschluß. Derartige den Substituenten X zugrundeliegende telechele Polyisobuten-Systeme und ihre Herstellung werden beispielsweise in der US-A 4 429 099 (7) beschrieben.

Im Rest der Formel Z bzw. Z' bezeichnen die Substituenten R¹⁰ und R¹¹ vorzugsweise Wasserstoff und/oder lineare oder verzweigte C₁- bis C₄-Alkylreste, insbesondere Methylreste. Die Verbindung I mit einem Rest Z bzw. Z', bei dem R¹⁰ = R¹¹ = Methyl ist, leitet sich von Bisphenol A [2,2-Bis-(4-hydroxyphenyl)propan] ab. Herstellungsbedingt können Verbindungen I mit einem Rest Z und Verbindungen I mit dem entsprechenden Rest Z' auch als Mischungen vorliegen.

Unter Hydrocarbylresten mit 1 bis 3000 bzw. 4 bis 3000 Kohlenstoffatomen für die Substituenten R¹, R², R³, R⁴, R⁵, R⁷ und R⁸ sollen hier reine Kohlenwasserstoffreste jeglicher Struktur verstanden werden, die definitionsgemäß auch noch durch ein oder mehrere Heteroatome aus der Gruppe O und S und/oder durch eine oder mehrere Gruppierungen NR⁶ unterbrochen sein können. Insbesondere sind Hydrocarbylreste Alkyl-, Alkenyl-, Cycloalkyl-, Aryl-, Alkylaryl-, Alkenylaryl- oder Arylalkyl-Reste.

Bei Unterbrechungen des Hydrocarbylrestes durch Gruppierungen NR⁶ sind auch solche Reste gemeint, bei denen am Ende die Gruppierungen NR⁶ formal in eine C-H-Bindung insertiert ist, also beispielsweise Substituenten R¹, R², R³, R⁴, R⁵, R⁷ oder R⁸ mit einer NH₂-Endgruppe. Derartige Hydrocarbylreste leiten sich z.B. von Polyaminen wie Ethylendiamin, Diethylentriamin, Triethylentetramin, Tetraethylenpentamin, etc. ab, bei denen eines der endständigen Stickstoffatome das N-Atom im Oxazin-Ring darstellt.

Der Ausdruck "Alkyl" umfasst geradkettige und verzweigte Alkylgruppen. Beispiele für Alkylgruppen sind neben den bereits oben genannten Methyl-, Ethyl-, n-Propyl-, iso-Propyl-, n-Butyl-, 2-Butyl-, sec.-Butyl- und tert.-Butyl-Resten insbesondere auch n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, n-Nonyl, n-Decyl, n-Dodecyl, n-Tridecyl, iso-Tridecyl, n-Tetradecyl (Myristyl), n-Hexadecyl (Palmityl), n-Octadecyl (Stearyl), und n-Eicosyl.

Beispiele für Alkenylreste sind Vinyl, 1-Propenyl, 2-Propenyl, Oleyl, Linolyl und Linolenyl.

Beispiele für Cylcoalkylreste sind C₅- bis C₇-Cycloalkylgruppen wie Cyclopentyl, Cyclohexyl und Cycloheptyl, welche noch durch Alkylgruppen, beispielsweise Methylreste, substituiert sein können.

Der Ausdruck "Aryl" umfasst einkernige, zweikernige, dreikernige und höherkernige aromatische Kohlenwasserstoffreste. Diese Arylreste können im Falle einer Substitution durch die beispielsweise vorgenannten Alkyl- und/oder Alkenylreste zu Alkylaryl- bzw. Alkenylarylresten noch 1, 2, 3, 4 oder 5, vorzugsweise 1, 2 oder 3 Substituenten tragen. Typische Beispiele sind Phenyl, Tolyl, Xylyl, Mesityl, Naphthyl, Fluorenyl, Anthracenyl, Phenanthrenyl, Naphthacenyl und Styryl. Ein typisches Beispiel für einen Arylalkylrest ist Benzyl.

Ist der längerkettige Hydrocarbylrest mit 4 bis 3000 Kohlenstoffatomen ein Polyisobutenylrest, kann er auf prinzipiell jedem gängigen und kommerziell erhältlichen Polyisobuten basieren, welches in geeigneter Weise in die Synthese der Tetrahydrobenzoxazine I eingebracht wird. Ein solches Polyisobuten besitzt vorzugsweise ein zahlenmittleres Molekulargewicht Mₙ von mindestens 200. Bevorzugt sind Polyisobutene mit einem zahlenmittleren Molekulargewicht Mₙ im Bereich von 200 bis 40.000, besonders bevorzugt von 500 bis 15.000, vor allem von 700 bis 7000, insbesondere von 900 bis 3000 und ganz besonders bevorzugt von 900 bis 1100. Unter den Begriff "Polyisobuten" fallen im Sinne der vorliegenden Erfindung auch oligomere Isobutene wie dimeres, trimeres, tetrameres, pentameres, hexameres und heptameres Isobuten.

Vorzugsweise leiten sich die in den erfindungsgemäß verwendeten Tetrahydrobenzoxazine I eingebauten Polyisobutenylreste von sogenanntem "hochreaktiven" Polyisobuten ab. "Hochreaktive" Polyisobutene unterscheiden sich von den "niedrigreaktiven" Polyisobutenen durch den Gehalt an terminal angeordneten Doppelbindungen. So enthalten hochreaktive Polyisobutene wenigstens 50 Mol-% terminal angeordnete Doppelbindungen, bezogen auf die Gesamtanzahl an Polyisobuten-Makromolekülen. Besonders bevorzugt sind Polyisobutene mit wenigstens 60 Mol-% und insbesondere mit wenigstens 80 Mol-% terminal angeordnete Doppelbindungen, bezogen auf die Gesamtanzahl an Polyisobuten-Makromolekülen. Bei den terminal angeordneten Doppelbindungen kann es sich sowohl um Vinyldoppelbindungen [-CH=_{C}(CH3)₂] (β-Olefin) als auch um Vinyliden-Doppelbindungen [-CH-C(=CH₂)-CH₃] (α-Olefin) handeln. Außerdem weisen die im Wesentlichen homopolymere Polyisobutenylreste einheitliche Polymergerüste auf. Darunter werden im Rahmen der vorliegenden Erfindung solche Polyisobuten-Systeme verstanden, die zu wenigstens 85 Gew.-%, vorzugsweise zu wenigstens 90 Gew.-% und besonders bevorzugt zu wenigstens 95 Gew.-% aus Isobuteneinheiten der Wiederholungeinheit [-CH₂C(CH₃)₂-] aufgebaut sind.

Ein weiteres bevorzugtes Merkmal der Polyisobutene, die den erfindungsgemäß verwendeten Tetrahydrobenzoxazine I zugrunde liegen können, ist, dass sie zu wenigstens 15 Gew.-%, insbesondere zu wenigstens 50 Gew.-%, vor allem zu wenigstens 80 Gew.-% mit einer tert.-Butylgruppe [-CH₂C(CH₃)₃] terminiert sind.

Weiterhin weisen die als Basis für die erfindungsgemäß verwendeten Tetrahydrobenzoxazine I vorzugsweise dienenden Polyisobutene vorzugsweise einen Polydispersitätsindex (PDI) von 1,05 bis 10, vorzugsweise von 1,05 bis 3,0, insbesondere von 1,05 bis 2,0 auf. Unter Polydispersität versteht man den Quotienten aus gewichtsmittlerem Molekulargewicht M_{w} und zahlenmittlerem Molekulargewicht Mₙ (PDI = M_{w}/Mₙ).

Unter als Basis für die erfindungsgemäß verwendeten Tetrahydrobenzoxazine I vorzugsweise dienenden Polyisobutenen werden im Sinne der vorliegenden Erfindungen auch alle durch kationische Polymerisation erhältlichen Polymerisate verstanden, die vorzugsweise wenigstens 60 Gew.-% Isobuten, besonders bevorzugt wenigstens 80 Gew.-%, vor allem wenigstens 90 Gew.-% und insbesondere wenigstens 95 Gew.-% Isobuten einpolymerisiert enthalten. Daneben können die Polyisobutene weitere Buten-isomere wie 1- oder 2-Buten sowie davon verschiedene olefinisch ungesättigte Monomere, die mit Isobuten unter kationischen Polymerisationsbedingungen copolymerisierbar sind, einpolymerisiert enthalten.

Als Isobuten-Einsatzstoffe für die Herstellung von Polyisobutenen, die als Basis für die erfindungsgemäß verwendeten Tetrahydrobenzoxazine I dienen können, eignen sich dementsprechend sowohl Isobuten selbst als auch isobutenhaltige C₄-Kohlenwasserstoffströme, beispielsweise C₄-Raffinate, C₄-Schnitte aus der Isobuten-Dehydrierung, C₄-Schnitte aus Steamcrackem, FCC-Crackem (FCC: Fluid Catalyzed Cracking), sofern sie weitgehend von darin enthaltenem 1,3-Butadien befreit sind. Besonders geeignete C₄-Kohlenwasserstoffströme enthalten in der Regel weniger als 500 ppm, vorzugsweise weniger als 200 ppm Butadien. Bei Einsatz von C₄-Schnitten als Einsatzmaterial übernehmen die von Isobuten verschiedenen Kohlenwasserstoffe die Rolle eines inerten Lösungsmittels.

Als mit Isobuten copolymerisierbare Monomere kommen Vinylaromaten wie Styrol und α-Methylstyrol, C₁-C₄-Alkylstyrole wie 2-, 3- und 4-Methylstyrol, sowie 4-tert.-Butylstyrol, Isoolefine mit 5 bis 10 C-Atomen wie 2-Methylbuten-1, 2-Methylpenten-1, 2-Methylhexen-1, 2-Ethylpenten-1, 2-Ethylhexen-1 und 2-Propylhepten-1 in Betracht.

Typische Polyisobutene, die als Basis für die erfindungsgemäß verwendeten Tetrahydrobenzoxazine I dienen können, sind beispielsweise die Glissopal®-Marken der BASF Aktiengesellschaft, z. B. Glissopal 550, Glissopal 1000 und Glissopal 2300, sowie die Oppanol®-Marken der BASF Aktiengesellschaft, z.B. Oppanol B10, B12 und B15.

Bespiele für im Sinne der vorliegenden Erfindung typische Tetrahydrobenzoxazine I mit einem Tetrahydrooxazin-Ring am Benzolkern sind die folgenden, wobei "PIB" einen von einem hochreaktiven Polyisobuten (Mₙ 1000) abgeleiteten Polyisobutenylrest und "PIB*" ein von einem hochreaktiven Polyisobuten (Mₙ 870) abgeleitetes Polyisobutenylen-Brückenglied bezeichnet:

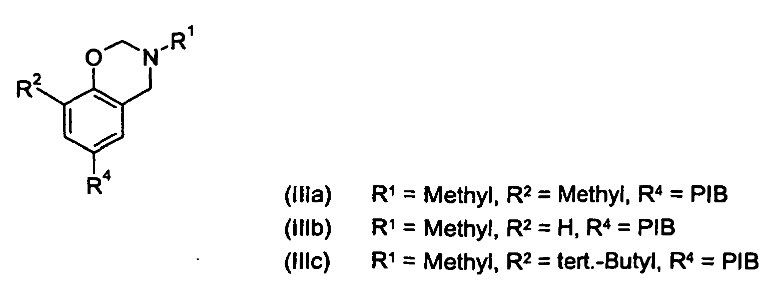

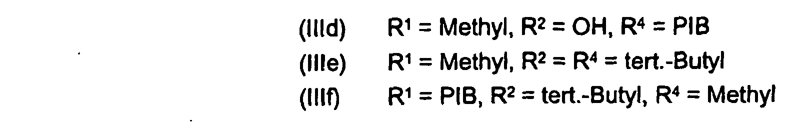

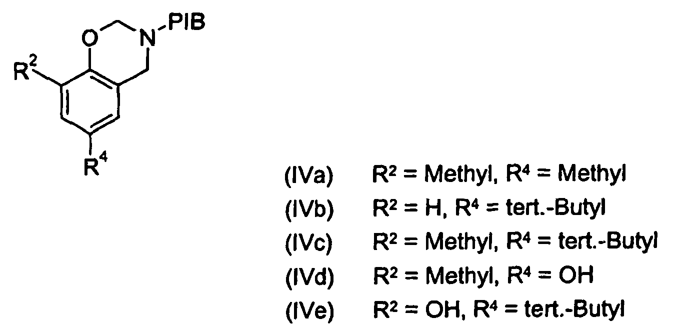

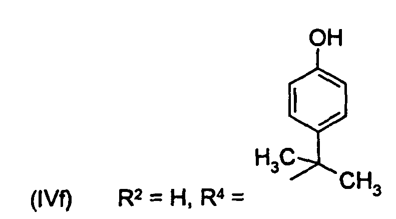

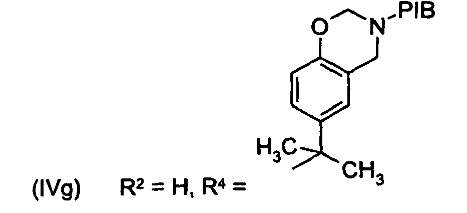

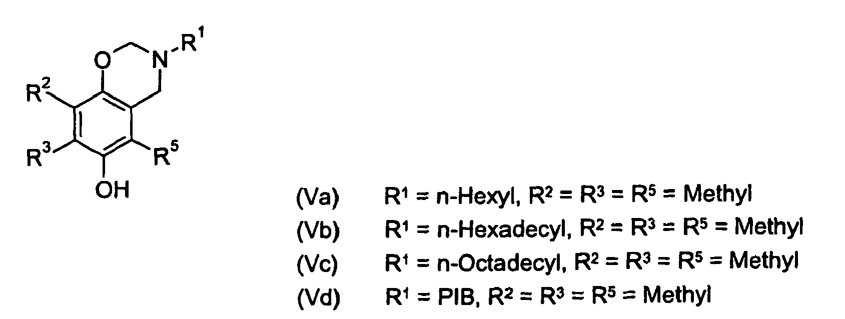

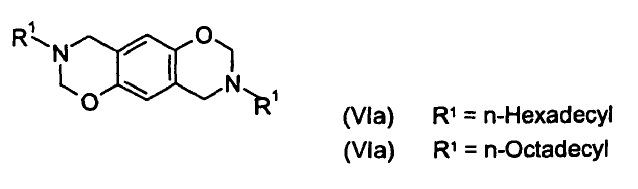

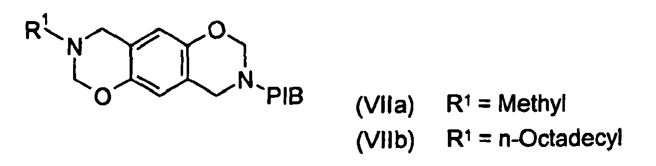

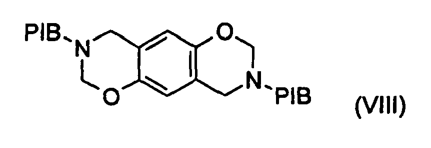

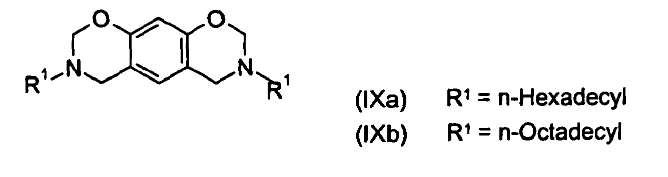

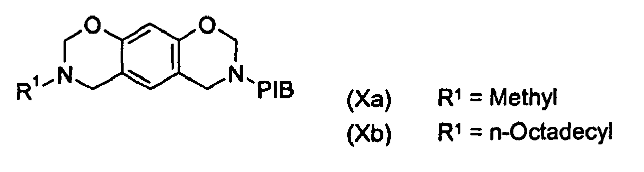

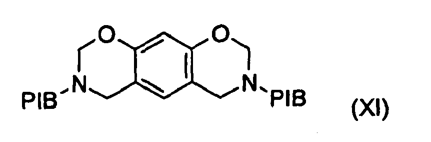

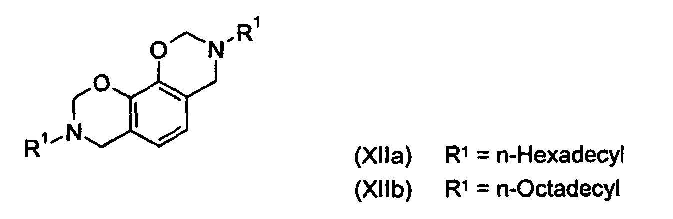

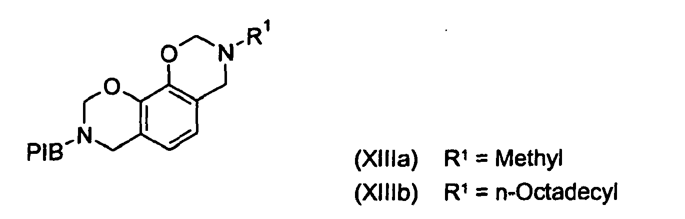

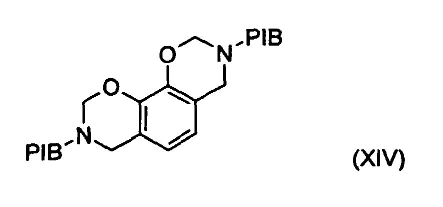

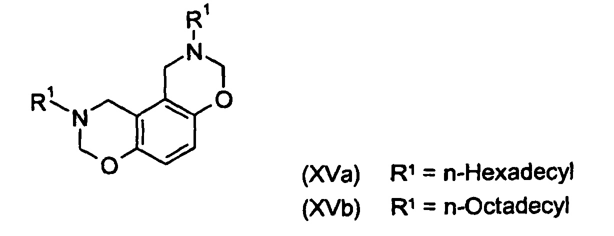

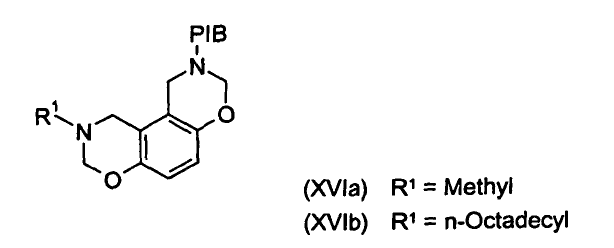

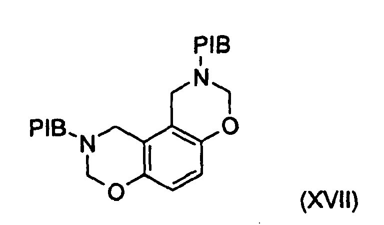

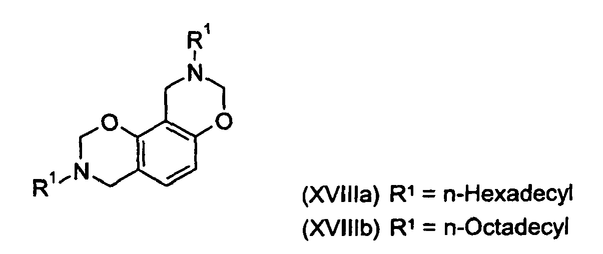

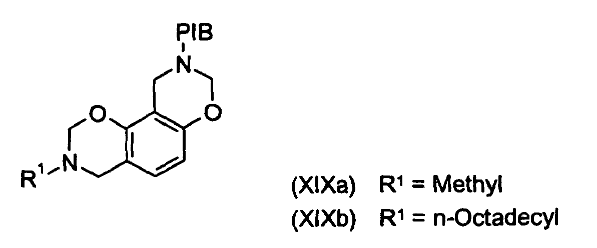

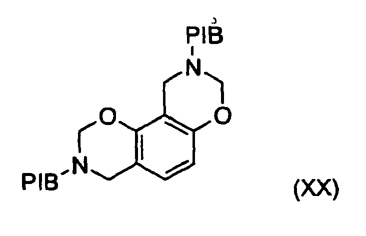

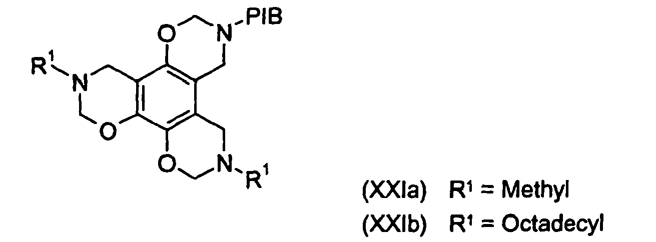

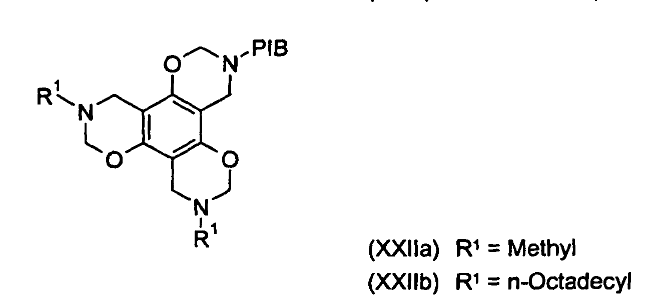

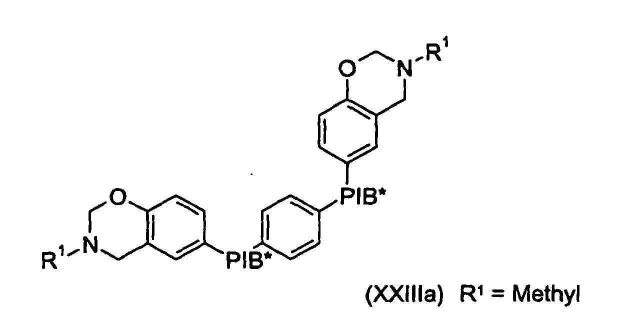

Herstellungsbedingt können auch Mischungen jeweils aus den Verbindungen VIa + XVa, VIb + XVb, VIIa + XVIa, VIIb + XVIb, VIII + XVII, IXa + XVIIIa, IXb + XVIIIb, Xa + XIXa, Xb + XIXb oder XI + XX auftreten und in dieser Form erfindungsgemäß verwendet werden.

In einer bevorzugten Ausführungsform verwendet man für die vorliegende Erfindung Tetrahydrobenzoxazine I, bei denen die Substituenten R³ und R⁴ oder R⁴ und R⁵ mit einer über den Substituenten R⁴ sauerstoff-angebundenen Teilstruktur -O-CH₂-NR⁷-CH₂- einen zweiten Tetrahydrooxazin-Ring ausbilden. Beispiele hierfür sind die oben aufgeführten Verbindungen VI bis XX.

Die beschriebenen Tetrahydrobenzoxazine I werden gemäß der vorliegenden Erfindung als Antioxidantien zur Stabilisierung von Mineralölprodukten und Kraftstoffen gegen die Einwirkung von Licht, Sauerstoff und Wärme verwendet. Hierunter ist insbesondere ihre Wirkungsweise als Antioxidantien im herkömmlichen Sinne zu verstehen. Dazu werden diese Verbindungen in das zu stabilisierende Material während oder nach dessen Herstellung eingearbeitet und möglichst homogen verteilt. Die Konzentration dieser Verbindungen in dem zu stabilisierenden Material beträgt in der Regel 0,0001 bis 5 Gew.-%, vorzugsweise 0,001 bis 5 Gew.-%, vor allem 0,01 bis 2 Gew.-%, insbesondere 0,05 bis 1 Gew.-%, bezogen auf das Material.

Unter Material sind Mineralölprodukte und Kraftstoffe, z.B. Dieselkraftstoff, Ottokraftstoff, Turbinenkraftstoff, Motor- oder Schmieröle, Getriebeöle und Schmierfette, zu verstehen.

Die beschriebenen Tetrahydrobenzoxazine I eignen sich in besonders vorteilhafter Weise als Antioxidantien in Turbinenkraftstoffen (jet fuels). Hierunter ist auch ihre Wirkungsweise als Antioxidantien im herkömmlichen Sinne zu verstehen. Insbesondere dienen sie über ihre Wirkungsweise als Antioxidantien zur Verbesserung der thermischen Stabilität von Turbinenkraftstoffen. Weiterhin verhindern sie insbesondere auch in ihrer Eigenschaft als Dispergatoren Ablagerungen im Kraftstoffsystem und/oder Verbrennungssystem von Turbinen. Turbinenkraftstoff werden vor allem zum Betreiben von Flugzeugturbinen eingesetzt.

Ein weiterer Gegenstand der vorliegenden Erfindung ist eine Turbinenkraftstoffzusammensetzung, die einen Turbinenkraftstoff (jet fuel) und wenigstens eines der beschriebenen Tetrahydrobenzoxazine I enthält.

Die erfindungsgemäße Turbinenkraftstoffzusammensetzung enthält eine Hauptmenge eines flüssigen Turbinenkraftstoffs, wobei es sich beispielsweise um einen in der zivilen oder militärischen Luftfahrt üblichen Turbinenkraftstoff handelt. Dazu zählen beispielsweise Kraftstoffe der Bezeichnung Jet Fuel A, Jet Fuel A-1, Jet Fuel B, Jet Fuel JP-4, JP-5, JP-7, JP-8 und JP-8+100. Jet A und Jet A-1 sind kommerziell erhältliche Turbinenkraftstoffspezifikationen auf Kerosinbasis. Die zugehörigen Normen sind ASTM D 1655 sowie DEF STAN 91-91. Jet B ist ein weiter geschnittener Kraftstoff auf Basis von Naphtha- und Kerosinfraktionen. JP-4 ist äquivalent zu Jet B. JP-5, JP-7, JP-8 und JP-8+100 sind militärische Turbinenkraftstoffe, wie sie beispielsweise von der Marine und Luftwaffe eingesetzt werden. Zum Teil bezeichnen diese Normen Formulierungen, die bereits weitere Additive, wie Korrosionsinhibitoren, Vereisungsinhibitoren, statische Dissipatoren, etc. enthalten.

Die beschriebenen Tetrahydrobenzoxazine I können dem Turbinenkraftstoff oder der Turbinenkraftstoffzusammensetzung einzeln, als Gemische und gegebenenfalls in Kombination mit weiteren an sich bekannten Zusatzstoffen zugegeben werden.

Geeignete Zusatzstoffe, die in der erfindungsgemäßen Turbinenkraftstoffzusammensetzung enthalten sein können, umfassen üblicherweise Detergenzien, Korrosionsinhibitoren, weitere Antioxidantien wie sterisch gehinderte tert.-Butylphenole, N-Butylphenylendiamine oder N,N'-Diphenylamin und Derivate hiervon, Metalldesaktivatoren wie N,N'-Disalicyliden-1,2-diaminopropan, Lösungsvermittler, Antistatika wie Stadis 450, Biocide, Anti-Icing-Mittel wie Diethylenglykolmethylether, sowie Mischungen der genannten Zusatzstoffe.

Im Rahmen der vorliegenden Erfindung bevorzugte Zusatzstoffe sind die nachfolgend aufgeführten speziellen Verbindungsklassen (A), (B) und (C):

Bevorzugte Zusatzstoffe (A) sind von Bernsteinsäureanhydrid abgeleitete Verbindungen mit langkettigen Kohlenwasserstoffresten mit in der Regel 15 bis 700, vor allem 30 bis 200 Kohlenstoffatomen. Diese Verbindungen können weitere funktionelle Gruppen aufweisen, die vorzugsweise ausgewählt sind unter Hydroxy-, Amino-, Amido- und/oder Imidogruppen. Bevorzugte Additive sind die entsprechenden Derivate von Polyalkenylbernsteinsäureanhydrid, welche z. B. durch Umsetzung von Polyalkenen mit Maleinsäureanhydrid auf thermischem Weg oder über die chlorierten Kohlenwasserstoffe erhältlich sind. Das zahlenmittlere Molekulargewicht der langkettigen Kohlenwasserstoffreste liegt vorzugsweise in einem Bereich von etwa 200 bis 10.000, besonders bevorzugt 400 bis 5000, insbesondere 600 bis 3000 und speziell 650 bis 2000. Vorzugsweise leiten sich diese langkettigen Kohlenwasserstoffreste von konventionellen und insbesondere von den zuvor genannten reaktiven Polyisobutenen ab. Von besonderem Interesse als Zusatzstoffe (A) sind die Derivate von Polyalkenylbemsteinsäureanhydriden mit Ammoniak, Monoaminen, Polyaminen, Monoalkoholen und Polyolen. Zur Derivatisierung bevorzugte Polyamine umfassen Ethylendiamin, Diethylentriamin, Triethylentetramin, Tetraethylenpentamin, Propylendiamin, etc. Geeignete Alkohole umfassen einwertige Alkohole, wie Ethanol, Allylalkohol, Dodecanol und Benzylalkohol, mehrwertige Alkohole, wie Ethylenglykol, Diethylenglykol, Propylenglykol, 1,2-Butandiol, Neopentylglykol, Glycerin, Trimethylolpropan, Erythrit, Pentaerythrit, Mannitol und Sorbitol.

Als Zusatzstoffe geeignete Bemsteinsäureanhydrid-Derivate (A) sind beispielsweise in der US 3 522 179, US 4 234 435, US 4 849 572, US 4 904 401, US 5 569 644 und US 6 165 235 beschrieben, worauf hier in vollem Umfang Bezug genommen wird.

Bevorzugte Zusatzstoffe (B) sind Polyalkenylthiophosphonatester. Der Polyalkenylrest diese Ester weist vorzugsweise ein zahlenmittleres Molekulargewicht im Bereich von etwa 300 bis 5000, besonders bevorzugt 400 bis 2000 und insbesondere 500 bis 1500 auf. Der Polyalkenylrest leitet sich vorzugsweise von Polyolefinen ab, wie sie zuvor bei der Komponente (A) als langkettiger Kohlenwasserstoffrest beschrieben wurden. Dabei handelt es sich speziell um Polyalkenylreste, die sich von konventionellen oder reaktiven Polyisobutenen ableiten. Geeignete Verfahren zur Herstellung geeigneter Polyalkenylthiophosphonatester durch Umsetzung eines Polyolefins mit einem Thiophosphorylierungsmittel sind z. B. in der US 5 725 611 beschrieben, worauf hier Bezug genommen wird.

Bevorzugte Zusatzstoffe (C) sind Mannich-Addukte. Derartige Addukte werden prinzipiell durch Mannich-Umsetzung von aromatischen Hydroxylverbindungen, insbesondere Phenol und Phenolderivaten, mit Aldehyden und Mono- oder Polyaminen erhalten. Vorzugsweise handelt es sich um die Umsetzungsprodukte von Polyisobuten-substituierten Phenolen mit Formaldehyd und Mono- oder Polyaminen wie Ethylendiamin, Diethylentriamin, Triethylentetramin, Tetraethylenpentamin, Dimethylaminopropylamin, etc. Geeignete Mannich-Addukte und Verfahren zu ihrer Herstellung sind z. B. in der US 5 876 468, EP-A 831 141, EP-A 1 233 990 und EP-A 1 226 188 beschrieben, worauf hier in vollem Umfang Bezug genommen wird.

Die erfindungsgemäße Turbinenkraftstoffzusammensetzung enthält die beschriebenen Tetrahydrobenzoxazine I in einer Menge von üblicherweise 0,0001 bis 1 Gew.-%, vorzugsweise 0,001 bis 0,5 Gew.-%, insbesondere 0,01 bis 0,2 Gew.-% und vor allem 0,01 bis 0,1 Gew.-%, bezogen auf die Gesamtmenge der Turbinenkraftstoffzusammensetzung.

Die Zusatzstoffe (A) bis (C) sowie gegebenenfalls weitere der zuvor genannten Zusatzstoffe können üblicherweise jeweils in Mengen von jeweils 0,0001 bis 1 Gew.-%, vorzugsweise 0,001 bis 0,6 Gew.-% und insbesondere 0,0015 bis 0,4 Gew.-%, bezogen auf die Gesamtmenge der Turbinenkraftstoffzusammensetzung, eingesetzt werden.

Ein weiterer Gegenstand der vorliegenden Erfindung ist ein Additivkonzentrat für Turbinenkraftstoffe (jet fuels), welches wenigstens eines der beschriebenen Tetrahydrobenzoxazine I sowie gegebenenfalls wenigstens ein Verdünnungsmittel sowie gegebenenfalls mindestens einen weiteren Zusatzstoff, der vorzugsweise unter den zuvor beschriebenen ausgewählt ist, enthält. In einer bevorzugten Ausführungsform enthält das erfindungsgemäße Additivkonzentrat, wie dann auch die erfindungsgemäße Turbinenkraftstoffzusammensetzung, einen oder mehrere Zusatzstoffe aus der Gruppe (A), (B) und (C), insbesondere auch Mischungen hieraus wie (A) + (B), (A) + (C), (B) + (C) und (A) + (B) + (C).

Geeignete Verdünnungsmittel sind beispielsweise bei der Erdölverarbeitung anfallende Fraktionen wie Kerosin, Naphtha oder Brightstock. Geeignet sind darüber hinaus aromatische und aliphatische Kohlenwasserstoffe wie Solvent Naphtha schwer, Solvesso® oder Shellsol® sowie Gemische dieser Lösungs- und Verdünnungsmittel.

Die beschriebenen Tetrahydrobenzoxazine I liegen im erfindungsgemäßen Additivkonzentrat vorzugsweise in einer Menge von 0,1 bis 100 Gew.-%, besonders bevorzugt von 1 bis 80 Gew.-% und insbesondere von 10 bis 70 Gew.-%, bezogen auf das Gesamtgewicht des Konzentrats, vor.

Die Erfindung wird anhand der folgenden Beispiele näher erläutert.

### Herstellungsbeispiele

### Beispiel 1: N,N-Di-(isobutoxymethyl)-N-methylamin

In einem 2 I-Vierhalskolben mit Wasserabscheider wurden 600 ml Isobutanol und 90 g Paraformaldehyd vorgelegt. Dann tropfte man 113 g einer 41 gew.-%igen wässrigen Methylamin-Lösung über 12 Minuten zu, wobei die Temperatur auf 44°C anstieg. Nach Zusatz von 200 ml Toluol wurde unter kräftigem Rückfluß erhitzt, wobei sich 126 ml Wasser abschieden. Niedersieder wurden am Rotationsverdampfer bei 60°C und 15 mbar abdestilliert. Man erhielt 244 g Produkt in Form einer hellen, klaren Flüssigkeit mit aminartigem Geruch.
1H-NMR (400 MHz, 16 Scans, CDCl₃):
δ = 4,23 ppm, 4H, CH₃-N[CH₂-O-CH₂-CH(CH₃)₂]₂
δ = 3,17 ppm, 4H, CH₃-N[CH₂-O-CH₂-CH(CH₃)₂]₂
δ = 2,55 ppm, 3H, CH₃-N[CH₂-O-CH₂-CH(CH₃)₂]₂
δ = 1,83 ppm, 2H, CH₃-N[CH₂-O-CH₂-CH(CH₃)₂]₂
δ = 0,91 ppm, 12H, CH₃-N[CH₂-O-CH₂-CH(CH₃)₂]₂

### Beispiel 2: Tetrahydrobenzoxazin der Formel Vc

In einem 500 ml-Vierhalskolben wurden 30 g Paraformaldehyd bei Raumtemperatur in 100 ml Isopropanol vorgelegt. 142 g aufgeschmolzenes n-Octadecylamin wurden rasch zugesetzt. Der Kolbeninhalt wurde bis zum Rückfluss erwärmt und 30 Minuten unter Rückfluss gerührt. 76 g Trimethylhydrochinon wurden in 3 Portionen innerhalb von 15 Minuten zugegeben. Es wurde 30 Minuten unter Rückfluss gerührt. Beim Abkühlen auf Raumtemperatur fiel ein Feststoff aus, der über eine D3-Filternutsche abgesaugt wurde. Der Filterrückstand wurde mehrmals mit Heptan gewaschen. Anschließend wurde der Rückstand über Nacht im Vakuumtrockenschrank bei 50°C und 100 mbar unter Stickstoffatmosphäre getrocknet. Man erhielt 186,4 g Produkt.
1H-NMR (400 MHz, 16 Scans, CDCl₃):
δ = 4,70 ppm, 2H, -CH₂-N(CH₃)-CH₂-O-
δ = 3,82 ppm, 2H, -CH₂-N(CH₃)-CH₂-O-

### Beispiel 3: Tetrahydrobenzoxazine der Formeln VIa + XVa

In einem 500 ml-Vierhalskolben wurden 30 g Paraformaldehyd bei Raumtemperatur in 100 ml Isopropanol vorgelegt. 142 g aufgeschmolzenes n-Octadecylamin wurden rasch zugesetzt. Der Kolbeninhalt wurde bis zum Rückfluss erwärmt und 30 Minuten unter Rückfluss gerührt. 27,5 g Hydrochinon wurden in 3 Portionen innerhalb von 15 Minuten zugegeben. Es wurde 30 Minuten unter Rückfluss gerührt. Beim Abkühlen auf Raumtemperatur fiel ein Feststoff aus, der über eine D3-Filternutsche abgesaugt wurde. Der Filterrückstand wurde mehrmals mit Heptan gewaschen. Anschließend wurde der Rückstand über Nacht im Vakuumtrockenschrank bei 50°C und 100 mbar unter Stickstoffatmosphäre getrocknet. Man erhielt 30 g Produkt in Form eines Gemisches aus VIa + XVa. - Das Filtrat wurde bei 140°C und 5mbar eingedampft. Man erhielt 145 g weiteres Produkt in Form eines Gemisches aus VIa + XVa.
1H-NMR (400 MHz, 16 Scans, CDCl₃):
VIa: δ = 4,70 ppm, 2H, -CH₂-N(C₁₈H₃₇)-CH₂-O-
   δ = 3,73 ppm, 2H, -CH₂-N(C₁₈H₃₇)-CH₂-O-
XVa: δ = 4,74 ppm, 2H, -CH₂-N(C₁₈H₃₇)-CH₂-O-
   ö = 3,86 ppm, 2H, -CH₂-N(C₁₈H₃₇)-CH₂-O-

Gemäß den Integralen der Spektren ergeben sich folgende Gew.-Verhältnisse:
VIa : XVa im Rückstand: 9 : 1
VIa : XVa im Filtrat: 1 : 1

### Beispiel 4: Tetrahydrobenzoxazin der Formeln IIIa

In einem 2000 ml-Vierhalskolben wurden 615 g 2-Methyl-4-polyisobutenylphenol (auf Basis des im Handel erhältlichen hochreaktiven Polyisobutens Glissopal® 1000 mit einem Mn von 1000) bei Raumtemperatur in 500 ml Toluol vorgelegt. 107 g N,N-Di-(isobutoxymethyl)-N-methylamin aus Beispiel 1 wurden rasch zugesetzt. Der Kolbeninhalt wurde 30 Minuten bei 80°C gerührt. Die Lösung wurde bei 120°C und 5 mbar eingedampft. Man erhielt 650 g Produkt in Form eines Öls, welches zur Abtrennung von als Nebenprodukt entstandenden geringen Mengen Amin/Aldehyd-Kondensationsprodukten noch mit Methanol gewaschen werden kann.
1H-NMR (400 MHz, 16 Scans, CDCl₃):
δ = 4,73 ppm, 2H, -CH₂-N(CH₃)-CH₂-O-
δ = 3,87 ppm, 2H, -CH₂-N(CH₃)-CH₂-O-

### Beispiel 5: Tetrahydrobenzoxazin der Formeln IIIe

In einem 1000 ml-Vierhalskolben wurden 206 g 2,4-Di-tert.-butylphenol bei Raumtemperatur in 400 ml Toluol vorgelegt. 203 g N,N-Di-(isobutoxymethyl)-N-methylamin aus Beispiel 1 wurden rasch zugesetzt. Der Kolbeninhalt wurde 60 Minuten bei 80°C gerührt. Die Lösung wurde bei 120°C und 5 mbar eingedampft. Man erhielt 260 g Produkt.
1H-NMR (400 MHz, 16 Scans, CD₂Cl₂):
δ = 4,73 ppm, 2H, -CH₂-N(CH₃)-CH₂-O-
δ = 3,90 ppm, 2H, -CH₂-N(CH₃)-CH₂-O-

### Beispiel 6: Tetrahydrobenzoxazine der Formeln VIII + XVII

In einem 2000 ml-Vierhalskolben wurden 24 g Paraformaldehyd bei Raumtemperatur in 200 ml Isobutanol vorgelegt. 615 g Kerocom® PIBA (im Handel erhältliche 65 gew.-%ige Lösung von Polyisobutenylamin auf Basis von hochreaktivem Polyisobuten, Mn = 1000, in Mihagol, einem n-Alkan-Gemisch) wurden rasch zugesetzt. Der Kolbeninhalt wurde zum Rückfluss erwärmt und 30 Minuten unter Rückfluss gerührt. Dann setzte man 200 ml Toluol zu und schleppte das gebildete Wasser aus. 22 g Hydrochinon wurden in 200 ml n-Butanol gelöst innerhalb von 15 Minuten zugegeben. Anschließend wurde 60 Minuten unter Rückfluss gerührt. Die Lösung wurde bei 140°C und 5 mbar eingedampft. Man erhielt 440 g Produkt.
1H-NMR (400 MHz, 16 Scans, CDCl₃):
VIII: ö = 4,70 ppm, 2H, -CH₂-N(PIB)-CH₂-O-
   ö = 3,73 ppm, 2H, -CH₂-N(PIB)-CH₂-O-
XVII: δ = 4,75 ppm, 2H, -CH₂-N(PIB)-CH₂-O-
   ö = 3,87 ppm, 2H, -CH₂-N(PIB)-CH₂-O-

Aus den Integralen der Spektren ergibt sich ein Gew.-Verhältnis von
VIII : XVII = 1 : 1,1.

### Beispiel 7: Tetrahydrobenzoxazin der Formeln IIIb

In einem 2000 ml-Vierhalskolben wurden 550 g 4-Polyisobutenylphenol (auf Basis des im Handel erhältlichen hochreaktiven Polyisobutens Glissopal® 1000 mit einem Mn von 1000) bei Raumtemperatur in 500 ml Toluol vorgelegt. 107 g N,N-Di-(isobutoxymethyl)-N-methylamin aus Beispiel 1 wurden rasch zugesetzt. Der Kolbeninhalt wurde 60 Minuten bei 80°C gerührt. Die Lösung wurde mit Methanol gewaschen und bei 120°C und 5 mbar eingedampft. Man erhielt 517 g Produkt.
1H-NMR (400 MHz, 16 Scans, CDCl₃):
δ = 4,72 ppm, 2H, -CH₂-N(CH₃)-CH₂-O-
ö = 3,90 ppm, 2H, -CH₂-N(CH₃)-CH₂-O-

### Beispiel 8: Tetrahydrobenzoxazin der Formeln Vd

In einem 4000 ml-Vierhalskolben wurden 52,8 g Paraformaldehyd bei Raumtemperatur in 400 ml Isopropanol vorgelegt. 1990 g Kerocom® PIBA (im Handel erhältliche 65 gew.-%ige Lösung von Polyisobutenylamin auf Basis von hochreaktivem Polyisobuten, Mn = 1000, in Mihagol, einem n-Alkan-Gemisch) wurden innerhalb von 78 Minuten zugesetzt. Der Kolbeninhalt wurde zum Rückfluss erwärmt und 40 Minuten unter Rückfluss gerührt. Danach wurden 138,1 g Trimethylhydrochinon bei 60 bis 80°C portionsweise zugegeben. Anschließend wurden 100 ml Toluol zugesetzt und es wurde 2 Stunden unter Rückfluß erwärmt. Nachdem nach Abkühlung auf Raumtemperatur die Isopropanol-Phase abgetrennt und verworden worden war, wurden 1000 ml Heptan zugesetzt. Die Lösung wurde mit Wasser und Methanol gewaschen und über Natriumsulfat getrocknet. Die trockene Lösung wurde bei 140°C und 5 mbar eingedampft. Man erhielt 1414 g Produkt.
1H-NMR (400 MHz, 16 Scans, CDCl₃):
δ = 4,70 ppm, 2H, -CH₂-N(PIB)-CH₂-O-
δ = 3,81 ppm, 2H, -CH₂-N(PIB)-CH₂-O-
δ = 2,12, 2,07 und 2,00 ppm, jeweils 3H, -CH₃ am Aromaten

### Beispiel 9: Tetrahydrobenzoxazin der Formeln XXIIIa

In einem 500 ml-Vierhalskolben wurden 110 g α,ω-Bis-(4-hydroxyphenyl)-polyisobuten mit einer mittenständigen p-Phenylen-Baueinheit (Mn = 2000), welches gemäß Dokument (7) hergestellt worden war, bei Raumtemperatur in 100 ml Toluol vorgelegt. 21,5 g N,N-Di-(isobutoxymethyl)-N-methylamin aus Beispiel 1 wurden rasch zugesetzt. Der Kolbeninhalt wurde 60 Minuten bei 80°C gerührt. Die Lösung wurde mit Methanol gewaschen und bei 80°C und 5 mbar eingedampft. Man erhielt 104 g Produkt.
1H-NMR (400 MHz, 16 Scans, CDCl₃):
δ = 4,71 ppm, 2H, -CH₂-N(CH₃)-CH₂-O-
δ = 3,88 ppm, 2H, -CH₂-N(CH₃)-CH₂-O-

### Beispiel 10: Tetrahydrobenzoxazin der Formeln IIIf

In einem 2000 ml-Vierhalskolben wurden 24 g Paraformaldehyd bei Raumtemperatur in 150 ml Isopropanol vorgelegt. 800 g Kerocom® PIBA (im Handel erhältliche 65 gew.-%ige Lösung von Polyisobutenylamin auf Basis von hochreaktivem Polyisobuten, Mn = 1000, in Mihagol, einem n-Alkan-Gemisch) wurden innerhalb von 20 Minuten zugesetzt. Der Kolbeninhalt wurde zum Rückfluss erwärmt und 30 Minuten unter Rückfluss gerührt. Danach wurden 66 g 2-tert.-Butyl-4-methylphenol, gelöst in 150 ml Isopropanol, zugetropft. Anschließend wurde noch 2 Stunden unter Rückfluß erwärmt. Nachdem nach Abkühlung auf Raumtemperatur die Isopropanol-Phase abgetrennt und verworden worden war, wurde die Lösung bei 140°C und 5 mbar eingedampft. Man erhielt 574 g Produkt.
1H-NMR (400 MHz, 16 Scans, CDCl₃):
δ = 4,80 ppm, 2H, -CH₂-N(PIB)-CH₂-O-
δ = 3,90 ppm, 2H, -CH₂-N(PIB)-CH₂-O-
δ = 2,21 ppm, 3H, -CH₃ am Aromaten

### Anwendungsbeispiele

### Beispiel 11: Überprüfung der thermischen Stabilität von Turbinenkraftstoff (jet fuel)

Es wurde ein Turbinenkraftstoff der Spezifikation Jet A nach ASTM D 1655 eingesetzt. Die Additivierung erfolgte mit jeweils 100 mg/l der Tetrahydrobenzoxazine bzw. Tetrahydrobenzoxazin-Gemische aus den Herstellungsbeispielen 4 und 6 bis 10.

In einem Dreihals-Glaskolben, der mit Rührer, Rückflusskühler und Thermometer versehen war, wurden zunächst bei Raumtemperatur 5 I Luft innerhalb 1 h durch 150 ml des zu untersuchenden Kraftstoffs geleitet. Anschließend wurde der Kraftstoff mit einem Ölbad auf 140°C erhitzt und weitere 5 h bei dieser Temperatur gerührt. Nach Abkühlen auf Raumtemperatur wurde die gesamte Kraftstoffmenge über einen 0,45 µm Membranfilter filtriert. Anschließend wurde der Filterrückstand nach 45 min Trocknen im Trockenschrank bei 115°C und anschließender 2-stündiger Trocknung unter Vakuum im Exsikkator gravimetrisch bestimmt:
Blindwert (ohne Additiv): 12,0 mg
erfindungsgemäß additiviert mit jeweils 100 mg/l der folgenden Verbindungen:

| | |
|---|---|
| Verbindung der Formel IIIa (Herstellungsbeispiel 4): | 4,6 mg |
| Verbindungen der Formeln VIII + XVII (Herstellungsbeispiel 6): | 2,1 mg |
| Verbindung der Formel IIIb (Herstellungsbeispiel 7): | 2,4 mg |
| Verbindung der Formel Vd (Herstellungsbeispiel 8): | 2,2 mg |
| Verbindung der Formel XXIIIa (Herstellungsbeispiel 9): | 1,5 mg |
| Verbindung der Formel IIIf (Herstellungsbeispiel 10): | 1,0 mg |

Durch den Einsatz des erfindungsgemäßen Additivs konnte die durch thermische Belastung des Turbinenkraftstoffs entstehende Partikelmenge deutlich reduziert werden.

### Beispiel 12: Verbesserung der thermischen Stabilität von Turbinenkraftstoff (jet fuel)

Es wurde ein Turbinenkraftstoff in Anlehnung an die Spezifikation Jet A nach ASTM D 1655 eingesetzt. Die Überprüfung der Thermostabilität erfolgte nach der JFTOT-Breakpoint-Methode nach ASTM D 3241. Bei dem nicht additivierten Turbinenkraftstoff wurde ein Wert von 240°C ermittelt. Mit Kraftstoffen, die mit jeweils 100 mg/l eines nachfolgend aufgeführten erfindungsgemäß verwendeten Additivs additiviert waren, wurden die folgenden Werte gemessen:

| | |
|---|---|
| Verbindung der Formel IIIa (Herstellungsbeispiel 4): | 280°C |
| Verbindung der Formel IIIb (Herstellungsbeispiel 7): | 270°C |
| Verbindung der Formel Vd (Herstellungsbeispiel 8): | 270°C |
| Verbindung der Formel XXIIIa (Herstellungsbeispiel 9): | 270°C |

### Beispiel 13: Überprüfung der Wasserverträglichkeit von Turbinenkraftstoff

Es wurden ein Turbinenkraftstoff der Spezifikation Jet A-1 nach DEF STAN 91-91 verwendet.

Gemäß DIN 51415 bzw. ASTM D 1094 wurde nach Zugabe von 100 mg/l des Produktes aus Herstellungsbeispiel 6 die Wasserverträglichkeit des Turbinenkraftstoffes und damit die unerwünschte Neigung, Emulsionen zu bilden, bestimmt. Hierzu wurden 80 ml des additivierten Turbinenkraftstoffes und 20 ml Wasser in definierter Weise intensiv geschüttelt. Danach erfolgte eine visuelle Bewertung der Phasentrennschichten nach jeweils 1, 5, 30 und 60 Minuten. Bereits 5 Minuten nach Wasserzugabe erhielt man eine vollständige Trennung von Kraftstoff und Wasser, es blieben keine Emulsionsanteile zurück.

Eine Wiederholung des Versuches mit 100 mg/l des Produktes aus Herstellungsbeispiel 7 führte zu dem gleichen Ergebnis.

### Beispiel 14: Überprüfung der Wasserabtrennungseigenschaften von Turbinenkraftstoff

Es wurde ein Turbinenkraftstoff der Spezifikation Jet A-1 nach DEF STAN 91-91 eingesetzt. Die Überprüfung der Neigung von Turbinenkraftstoffen bezüglich ihrer Wasserabtrennungseigenschaften erfolgte nach ASTM D 3948 ("MSEP"-Test). Charakteristisch für diese Messungen ist die Verwendung eines Standard-Koaleszierfilters mit abschließender Trübungsmessung der Kraftstoffphase. Bei der Messung wurden die erfindungsgemäß verwendeten Additive in Kombination mit dem Antioxidans 2,6-Di-tert.-butyl-4-methylphenol ("BHT") und dem Metalldeaktivator N,N'-Disalicyliden-1,2-diaminopropan in einem hierfür üblichen Lösungsmittel geprüft. Die Dosierung der erfindungsgemäß verwendeten Additive betrug jeweils 215 mg/l (bezogen auf deren 100%igen Wirkstoffgehalt). Es wurden folgende Benotungen für das Trübungsverhalten ermittelt [relative Bewertungsskala von 0 (schlechteste Note) bis 100 (beste Note)]:

| | |
|---|---|
| Blindwert (ohne Additiv): | 99 |
| Verbindung der Formel IIIa (Herstellungsbeispiel 4): | 95 |
| Verbindung der Formel IIIb (Herstellungsbeispiel 7): | 99 |
| Verbindung der Formel Vd (Herstellungsbeispiel 8): | 94 |

Es traten keine Verschlechterungen im Vergleich mit nicht-additiviertem Turbinenkraftstoff auf.

## Patentansprüche

1. Verwendung von Tetrahydrobenzoxazinen der allgemeinen Formel I 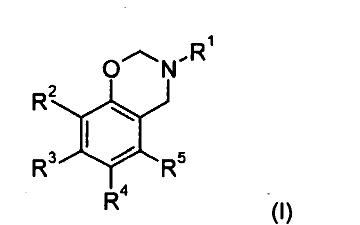 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und
die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen stehen, welche durch ein oder mehrere Heteroatome aus der Gruppe O und S und/oder durch eine oder mehrere Gruppierungen NR⁶ unterbrochen sein können,
wobei der Substituent R⁴ auch für einen Rest der Formel Y stehen kann 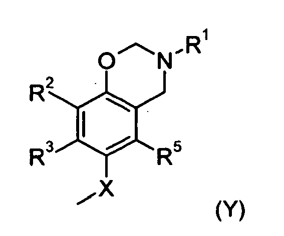 in dem die Substituenten R¹, R², R³ und R⁵ die vorgenannten Bedeutungen haben und der Substituent X ein Kohlenwasserstoff-Brückenglied bezeichnet, welches aus einem oder mehreren Isobuten-Einheiten besteht oder ein oder mehrere Isobuten-Einheiten enthält, oder
wobei der Substituent R⁴ auch für einen Rest der Formel Z oder Z' stehen kann 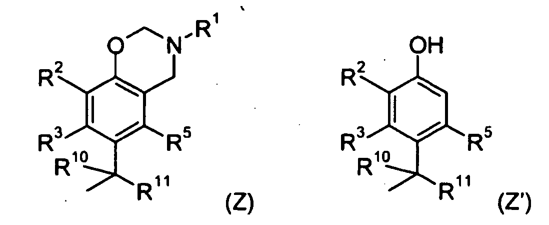 in denen die Substituenten R¹, R², R³ und R⁵ die vorgenannten Bedeutungen haben und die Substituenten R¹⁰ und R¹¹ gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR⁷-CH₂- auch einen zweiten Tetrahydrooxazin-Ring oder die Substituenten R² und R³ und R⁴ und R⁵ mit den an den Benzolkern angebundenen Teilstrukturen -O-CH₂-NR⁷-CH₂- und -O-CH₂-NR⁸-CH₂- auch einen zweiten und einen dritten Tetrahydrooxazin-Ring ausbilden können,
wobei R⁷ und R⁸ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können,
mit der Maßgabe, dass mindestens einer der Substituenten R¹, R², R³, R⁴, R⁵, R⁷ oder R⁸ 4 bis 3000 Kohlenstoffatome aufweist und die übrigen Substituenten aus der Gruppe R¹, R², R³, R⁴, R⁵, R⁷ und R⁸, wenn sie für Hydrocarbylreste stehen, jeweils 1 bis 20 Kohlenstoffatome aufweisen,
als Antioxidantien zur Stabilisierung von Mineralölprodukten und Kraftstoffen gegen die Einwirkung von Licht, Sauerstoff und Wärme.

2. Verwendung von Tetrahydrobenzoxazinen I nach Anspruch 1, bei denen mindestens einer der Substituenten R¹, R², R³, R⁴, R⁵, R⁷ oder R⁸ einen PolyisobutenylRest mit einem zahlenmittleren Molekulargewicht Mₙ von 200 bis 40.000 darstellt.

3. Verwendung von Tetrahydrobenzoxazinen I nach Anspruch 2, bei denen ein oder zwei Polyisobutenyl-Reste mit einem zahlenmittleren Molekulargewicht Mₙ von 200 bis 40.000 im Molekül als Substituent R¹ und/oder R² und/oder R⁴ und/oder R⁷ und/oder R⁸ auftreten.

4. Verwendung von Tetrahydrobenzoxazinen I nach den Ansprüchen 1 bis 3, bei denen die übrigen Substituenten aus der Gruppe R¹, R², R³, R⁴, R⁵, R⁷ und R⁸, die nicht für Substituenten mit 4 bis 3000 Kohlenstoffatomen bzw. für Polyisobutenyl-Reste mit einem zahlenmittleren Molekulargewicht Mₙ von 200 bis 40.000 stehen, unabhängig voneinander Wasserstoffatome, Hydroxylgruppen oder, wenn sie für Hydrocarbylreste stehen, lineare oder verzweigte C₁- bis C₄-Alkylreste bezeichnen.

5. Verwendung von Tetrahydrobenzoxazinen I nach den Ansprüchen 1 bis 4, bei denen die Substituenten R³ und R⁴ oder R⁴ und R⁵ mit einer über den Substituenten R⁴ sauerstoff-angebundenen Teilstruktur -O-CH₂-NR⁷-CH₂- einen zweiten Tetrahydrooxazin-Ring ausbilden.

6. Verwendung von Tetrahydrobenzoxazinen I nach den Ansprüchen 1 bis 5 als Antioxidantien in Turbinenkraftstoffen (jet fuels).

7. Verwendung von Tetrahydrobenzoxazinen I nach Anspruch 6 als Antioxidantien zur Verbesserung der thermischen Stabilität von Turbinenkraftstoffen.

8. Turbinenkraftstoffzusammensetzung, enthaltend einen Turbinenkraftstoff (jet fuel) und wenigstens ein Tetrahydrobenzoxazin der allgemeinen Formel I, wie in einem der Ansprüche 1 bis 5 definiert.

9. Additivkonzentrat für Turbinenkraftstoffe (jet fuels), enthaltend wenigstens ein Tetrahydrobenzoxazin der allgemeinen Formel I, wie in einem der Ansprüche 1 bis 5 definiert, sowie gegebenenfalls wenigstens ein Verdünnungsmittel und gegebenenfalls wenigstens einen Zusatzstoff.

## Claims

1. The use of tetrahydrobenzoxazines of the general formula I 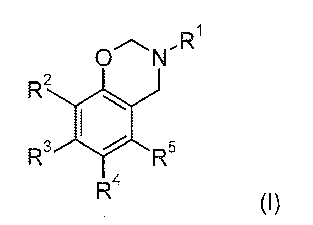 in which the substituent R¹ represents a hydrocarbyl radical which has from 1 to 3000 carbon atoms and may be interrupted by one or more heteroatoms from the group of O and S and/or by one or more NR⁶ moieties,
where R⁶ represents a hydrogen atom or a C₁- to C₄-alkyl radical, and
the substituents R², R³, R⁴ and R⁵ are each independently hydrogen atoms, hydroxyl groups or hydrocarbyl radicals which have in each case from 1 to 3000 carbon atoms and may be interrupted by one or more heteroatoms from the group of O and S and/or by one or more NR⁶ moieties,
where the substituent R⁴ may also be a radical of the formula Y 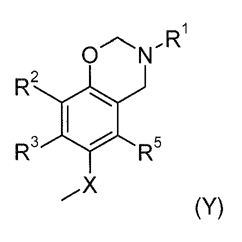 in which the substituents R¹, R², R³ and R⁵ are each as defined above and the substituent X is a hydrocarbon bridging element which consists of one or more isobutene units or comprises one or more isobutene units, or
where the substituent R⁴ may also be a radical of the formula Z or Z' 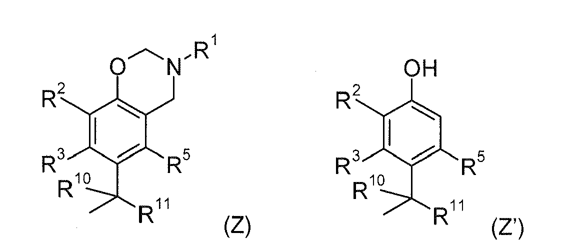 in which the substituents R¹, R², R³ and R⁵ are each as defined above and the substituents R¹⁰ and R¹¹ may be the same or different and represent hydrogen or a C₁- to C₁₀-alkyl radical,
and in which the substituents R² and R³ or R³ and R⁴ or R⁴ and R⁵, together with the part-structure -O-CH₂-NR⁷-CH₂- attached to the benzene ring, may also form a second tetrahydrooxazine ring, or the substituents R² and R³ and R⁴ and R⁵, together with the part-structures -O-CH₂-NR⁷-CH₂- and -O-CH₂-NR⁸-CH₂- attached to the benzene ring, may also form a second and a third tetrahydrooxazine ring,
where R⁷ and R⁸ are each independently hydrocarbyl radicals which have in each case from 1 to 3000 carbon atoms and may be interrupted by one or more heteroatoms from the group of O and S and/or by one or more NR⁶ moieties,
with the proviso that at least one of the substituents R¹, R², R³, R⁴, R⁵, R⁷ or R⁸ has from 4 to 3000 carbon atoms and the remaining substituents from the group of R¹, R², R³, R⁴, R⁵, R⁷ and R⁸, when they are hydrocarbyl radicals, each have from 1 to 20 carbon atoms,
as antioxidants for stabilizing mineral oil products and fuels against the action of light, oxygen and heat.

2. The use of tetrahydrobenzoxazines I according to claim 1, in which at least one of the substituents R¹, R², R³, R⁴, R⁵, R⁷ or R⁸ is a polyisobutenyl radical having a number-average molecular weight Mₙ of from 200 to 40 000.

3. The use of tetrahydrobenzoxazines I according to claim 2, in which one or two polyisobutenyl radicals having a number-average molecular weight Mₙ of from 200 to 40 000 occur in the molecule as substituent R¹ and/or R² and/or R⁴ and/or R⁷ and/or R⁸.

4. The use of tetrahydrobenzoxazines I according to claims 1 to 3, in which the remaining substituents from the group of R¹, R², R³, R⁴, R⁵, R⁷ and R⁸ which are not substituents having from 4 to 3000 carbon atoms or polyisobutenyl radicals having a number-average molecular weight Mₙ of from 200 to 40 000 each independently represent hydrogen atoms, hydroxyl groups or, when they are hydrocarbyl radicals, linear or branched C₁- to C₄-alkyl radicals.

5. The use of tetrahydrobenzoxazines I according to claims 1 to 4, in which the substituents R³ and R⁴ or R⁴ and R⁵, together with a part-structure O-CH₂-NR⁷-CH₂-oxygen-attached via the substituent R⁴ form a second tetrahydrooxazine ring.

6. The use of tetrahydrobenzoxazines I according to claims 1 to 5 as antioxidants in turbine fuels (jet fuels).

7. The use of tetrahydrobenzoxazines I according to claim 6 as antioxidants for improving the thermal stability of turbine fuels.

8. A turbine fuel composition comprising a turbine fuel (jet fuel) and at least one tetrahydrobenzoxazine of the general formula I as defined in any of claims 1 to 5.

9. An additive concentrate for turbine fuels (jet fuels), comprising at least one tetrahydrobenzoxazine of the general formula I as defined in any of claims 1 to 5 and also optionally at least one diluent and optionally at least one additive.

## Revendications

1. Utilisation de tétrahydrobenzoxazines de formule générale I 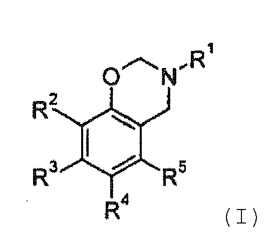 dans laquelle le substituant R¹ représente un radical hydrocarbyle ayant de 1 à 3 000 atomes de carbone, qui peut être interrompu par un ou plusieurs hétéroatomes choisis dans l'ensemble constitué par les atomes d'oxygène et de soufre et/ou par un ou plusieurs groupements NR⁶,
R⁶ étant un atome d'hydrogène ou un radical alkyle en C₁-C₄, et
les substituants R², R³, R⁴ et R⁵ représentent, indépendamment les uns des autres, des groupes hydroxy ou des radicaux hydrocarbyle ayant chacun de 1 à 3 000 atomes de carbone, qui peuvent être interrompus par un ou plusieurs hétéroatomes choisis dans l'ensemble constitué par les atomes d'oxygène et de soufre et/ou par un ou plusieurs groupements NR⁶,
le substituant R⁴ pouvant également représenter un radical de formule Y 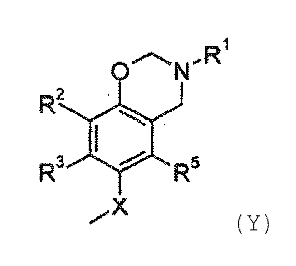 dans lequel les substituants R¹, R², R³ et R⁵ ont les significations données précédemment et le substituant X représente un chaînon pontant hydrocarboné, qui consiste en une ou plusieurs unités isobutène ou contient une ou plusieurs unités isobutène, ou
le substituant R⁴ pouvant également représenter un radical de formule Z ou Z' 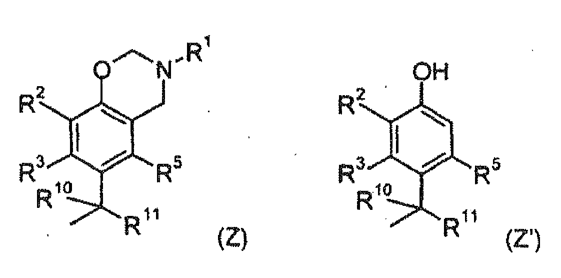 formules dans lesquelles les substituants R¹, R², R³ et R⁵ ont les significations données précédemment et les substituants R¹⁰ et R¹¹ peuvent être identiques ou différents et représentent un atome d'hydrogène ou un radical alkyle en C₁-C₁₀,
et dans laquelle les substituants R² et R³ ou R³ et R⁴ ou R⁴ et R⁵ peuvent également former avec la structure partielle -O-CH₂-NR⁷-CH₂- liée au noyau benzénique un deuxième cycle tétrahydro-oxazine ou les substituants R² et R³ et R⁴ et R⁵ peuvent également former avec les structures partielles -O-CH₂-NR⁷-CH₂- et -O-CH₂-NR⁸-CH₂-liées au noyau benzénique un deuxième et un troisième cycle tétrahydro-oxazine,
R⁷ et R⁸ représentant, indépendamment l'un de l'autre, des radicaux hydrocarbyle ayant chacun de 1 à 3 000 atomes de carbone, qui peuvent être interrompus par un ou plusieurs hétéroatomes choisis dans le groupe constitué par les atomes d'oxygène et de soufre et/ou par un ou plusieurs groupements NR⁶,
étant entendu qu'au moins l'un des substituants R¹, R², R³, R⁴, R⁵, R⁷ ou R⁸ a de 4 à 3 000 atomes de carbone et les autres substituants dans l'ensemble constitué par R¹, R², R³, R⁴, R⁵, R⁷ et R⁸, lorsqu'ils représentent des radicaux hydrocarbyle, ont chacun de 1 à 20 atomes de carbone,
en tant qu'antioxydants pour la stabilisation de produits de type huile minérale et de carburants vis-à-vis de l'action de la lumière, de l'oxygène et de la chaleur.

2. Utilisation de tétrahydrobenzoxazines I selon la revendication 1, dans lesquelles au moins l'un des substituants R¹, R², R³, R⁴, R⁵, R⁷ ou R⁸ représente un reste polyisobutényle ayant une masse moléculaire moyenne Mₙ de 200 à 40 000.

3. Utilisation de tétrahydrobenzoxazines I selon la revendication 2, dans lesquelles un ou deux radicaux polyisobutényle ayant une masse moléculaire moyenne en nombre Mₙ de 200 à 40 000 apparaissent dans la molécule en tant que substituant(s) R¹ et/ou R² et/ou R⁴ et/ou R⁷ et/ou R⁸.

4. Utilisation de tétrahydrobenzoxazines I selon les revendications 1 à 3, dans lesquelles les autres substituants dans l'ensemble constitué par R¹, R², R³, R⁴, R⁵, R⁷ et R⁸ qui ne représentent pas des substituants ayant de 4 à 3 000 atomes de carbone ou des radicaux polyisobutényle ayant une masse moléculaire moyenne en nombre Mₙ de 200 à 40 000 représentent, indépendamment les uns des autres, des atomes d'hydrogène, des groupes hydroxy ou, lorsqu'ils représentent des radicaux hydrocarbyle, désignent des radicaux alkyle en C₁-C₄ linéaires ou ramifiés.

5. Utilisation de tétrahydrobenzoxazines I selon les revendications 1 à 4, dans lesquelles les substituants R³ et R⁴ ou R⁴ et R⁵ forment avec une structure partielle -O-CH₂-NR⁷-CH₂- liée à l'oxygène par le substituant R⁴ un deuxième cycle tétrahydro-oxazine.

6. Utilisation de tétrahydrobenzoxazines I selon les revendications 1 à 5, en tant qu'antioxydants dans des carburants pour moteurs à réaction (jet fuels).

7. Utilisation de tétrahydrobenzoxazines I selon la revendication 6, en tant qu'antioxydants pour l'amélioration de la stabilité thermique de carburants pour moteurs à réaction.

8. Composition de carburant pour moteurs en réaction, contenant un carburant pour moteurs à réaction (jet fuel) et au moins une tétrahydrobenzoxazine de formule générale I, telle que définie dans l'une quelconque des revendications 1 à 5.

9. Concentré d'additif pour carburants pour moteurs à réaction (jet fuels), contenant au moins une tétrahydrobenzoxazine de formule générale I, telle que définie dans l'une quelconque des revendications 1 à 5, ainsi qu'éventuellement au moins un diluant et éventuellement au moins un additif.
